(19) Europäisches Patentamt
European Patent Office
Office européen des brevets

(11) EP 1 195 154 A2

(12) **EUROPÄISCHE PATENTANMELDUNG**

(43) Veröffentlichungstag:
**10.04.2002 Patentblatt 2002/15**

(51) Int Cl.[7]: **A61K 7/00**, A61K 7/48, B01F 17/00

(21) Anmeldenummer: **01123637.9**

(22) Anmeldetag: **02.10.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **04.10.2000 DE 10049066**

(71) Anmelder: **Beiersdorf AG**
**20253 Hamburg (DE)**

(72) Erfinder:
- **Bleckmann, Andreas**
  **22926 Ahrensburg (DE)**
- **Kröpke, Rainer**
  **22869 Schenefeld (DE)**
- **Syskowski, Boris**
  **2233 Hamburg (DE)**

(54) **Zubereitungen vom Emulsionstyp W/O mit erhöhtem Wassergehalt enthaltend Polyetherpolyester**

(57) Wasser-in-Öl- Emulsionen

(a) eines Gehaltes an Wasser und gegebenenfalls wasserlöslichen Substanzen von insgesamt mindestens 70 Gew.-%, und eines Gehaltes an Lipiden, Emulgatoren und lipophilen Bestandteilen von insgesamt höchstens 20% jeweils bezogen auf das Gesamtgewicht der Zubereitungen,
(b) mit einer Lipidphase deren Gesamtpolarität zwischen 20 und 45 mN/m liegt
(c) enthaltend wenigstens eine grenzflächenaktive Substanz, gewählt aus der Gruppe der Substanzen der allgemeinen Formel (I)

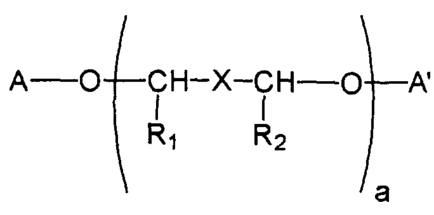

,

- wobei A und A' gleiche oder verschiedene organische Reste, gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema

EP 1 195 154 A2

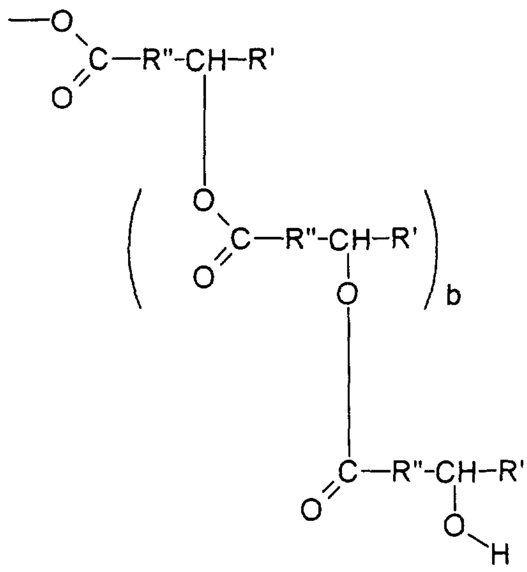

,

wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann,

- a eine Zahl von 1 bis 100, vorzugsweise 2 bis 60, insbesondere 5 bis 40 darstellt,
- X eine Einfachbindung oder die Gruppe

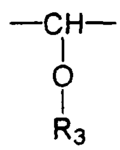

- darstellt,
- $R_1$ und $R_2$ unabhängig voneinander aus der Gruppe H, Methyl gewählt werden,
- $R_3$ gewählt wird aus der Gruppe H, sowie der verzweigten und unverzweigten, gesättigten und ungesättigten Alkyl- und Acylreste mit 1 - 20 Kohlenstoffatomen,

(d) enthaltend wenigstens eine Substanz, gewählt aus der Gruppe der anionischen und/oder amphoteren Polymere

## Beschreibung

Die vorliegende Erfindung betrifft kosmetische und dermatologische Zubereitungen, insbesondere solche vom Typ Wasser-in-Öl, Verfahren zu ihrer Herstellung sowie ihre Verwendung für kosmetische und medizinische Zwecke.

Die vorliegende Erfindung betrifft in einer besonderen Ausführungsform kosmetische oder pharmazeutische Zubereitungen mit verbessertem Hautgefühl, vermindertem Klebrigkeitsgefühl, Verfahren zu ihrer Herstellung sowie die Verwendung von Wirkstoffen zur Herabminderung des Klebrigkeitsgefühles kosmetischer Zubereitungen.

Die menschliche Haut übt als größtes Organ des Menschen zahlreiche lebenswichtige Funktionen aus. Mit durchschnittlich etwa 2 $m^2$ Oberfläche beim Erwachsenen kommt ihr eine herausragende Rolle als Schutz- und Sinnesorgan zu. Aufgabe dieses Organs ist es, mechanische, thermische, aktinische, chemische und biologische Reize zu vermitteln und abzuwehren. Außerdem kommt ihr eine bedeutende Rolle als Regulations- und Zielorgan im menschlichen Stoffwechsel zu.

Unter kosmetischer Hautpflege ist in erster Linie zu verstehen, die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) zu stärken oder wiederherzustellen sowie ihre Hornschicht bei aufgetretenen Schäden in ihrem natürlichen Regenerationsvermögen zu unterstützen.

Werden die Barriereeigenschaften der Haut gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es femer, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (OIW-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Natürlich ist dem Fachmann eine Vielzahl von Möglichkeiten bekannt, stabile W/O-Zubereitungen zur kosmetischen oder dermatologischen Anwendung zu formulieren, beispielsweise in Form von Cremes und Salben, die im Bereich von Raum- bis Hauttemperatur streichfähig sind, oder als Lotionen und Milche, die in diesem Temperaturbereich eher fließfähig sind. Der Stand der Technik kennt allerdings nur wenige Formulierungen, die so dünnflüssig sind, daß sie beispielsweise sprühbar wären.

Zudem haben dünnflüssige Zubereitungen des Standes der Technik häufig den Nachteil, daß sie instabil, auf einen engen Anwendungsbereich oder eine begrenzte Einsatzstoffauswahl begrenzt sind. Dünnflüssige Produkte, in denen beispielsweise stark polare Öle - wie die in handelsüblichen Produkten sonst häufig verwendeten Pflanzenöle - ausreichend stabilisiert sind, gibt es daher zur Zeit auf dem Markt nicht.

Unter dem Begriff "Viskosität" versteht man die Eigenschaft einer Flüssigkeit, der gegenseitigen laminaren Verschiebung zweier benachbarter Schichten einen Widerstand (Zähigkeit, innere Reibung) entgegenzusetzen. Man definiert heute diese sogenannte dynamische Viskosität nach $\eta=\tau/D$ als das Verhältnis der Schubspannung zum Geschwindigkeitsgradienten senkrecht zur Strömungsrichtung. Für newtonsche Flüssigkeiten ist $\eta$ bei gegebener Temperatur eine Stoffkonstante mit der SI-Einheit Pascalsekunde (Pa·s).

Der Quotient $\nu=\eta/\rho$ aus der dynamischen Viskosität $\eta$ und der Dichte $\rho$ der Flüssigkeit wird als kinematische Viskosität $\nu$ bezeichnet und in der SI-Einheit $m^2/s$ angegeben.

Als Fluidität ($\varphi$) bezeichnet man den Kehrwert der Viskosität ($\varphi=1/\eta$). Bei Salben und dergleichen wird der Gebrauchswert unter anderem mitbestimmt von der sogenannten Zügigkeit. Unter der Zügigkeit einer Salbe oder Salbengrundlage oder dergleichen versteht man deren Eigenschaft, beim Abstechen verschieden lange Fäden zu ziehen; dementsprechend unterscheidet man kurz- und langzügige Stoffe.

Während die graphische Darstellung des Fließverhaltens newtonscher Flüssigkeiten bei gegebener Temperatur eine Gerade ergibt, zeigen sich bei den sogenannten nichtnewtonschen Flüssigkeiten in Abhängigkeit vom jeweiligen Geschwindigkeitsgefälle D (Schergeschwindigkeit $\dot{\gamma}$) bzw. der Schubspannung $\tau$ oft erhebliche Abweichungen.

In diesen Fällen läßt sich die sogenannte scheinbare Viskosität bestimmen, die zwar nicht der Newtonschen Gleichung gehorcht, aus der sich jedoch durch graphische Verfahren die wahren Viskositätswerte ermitteln lassen.

Die Fallkörperviskosimetrie ist lediglich zur Untersuchung newtonscher Flüssigkeiten sowie von Gasen geeignet. Sie basiert auf dem Stokes-Gesetz, nach dem für das Fallen einer Kugel durch eine sie umströmende Flüssigkeit die dynamische Viskosität η aus

$$\eta = \frac{2r^2(\rho_K - \rho_{Fl})\cdot g}{9\cdot v}$$

bestimmbar ist, wobei
r = Radius der Kugel, v = Fallgeschwindigkeit, $\rho_K$ = Dichte der Kugel, $\rho_{Fl}$ = Dichte der Flüssigkeit und g = Fallbeschleunigung.

W/O-Emulsionen mit hohem Wassergehalt und einer geringen Viskosität, die darüberhinaus eine Lagerstabilität aufweisen, wie sie für marktgängige Produkte gefordert wird, sind nach dem Stand der Technik nur sehr aufwendig zu formulieren. Dementsprechend ist das Angebot an derartigen Formulierungen äußerst gering. Gleichwohl könnten derartige Formulierungen dem Verbraucher bisher nicht gekannte kosmetische Leistungen bieten.

Eine Aufgabe der vorliegenden Erfindung war es, Zubereitungen zur Verfügung zu stellen, welche eine sehr geringe Viskosität haben und nicht die Nachteile des Standes der Technik aufweisen.

Eine weitere Aufgabe der vorliegenden Erfindung war es, Zubereitungen zur Verfügung zustellen, welche mit einem hohen Gehalt an wasserlöslichen und/oder wassermischbaren Substanzen mit kosmetischer oder dermatologischer Wirksamkeit beladen werden können, ohne daß die galenische Qualität oder andere Eigenschaften der Zubereitungen beeinträchtigt wären.

Es ist zwar bekannt, durch Hinzufügen bestimmter Substanzen, beispielsweise einiger ausgewählter Puderrohstoffe, insbesondere Talkum, dieses Klebrigkeitsgefühl oder auch Schmierigkeitsgefühl zu reduzieren. Davon abgesehen, daß dieses nur selten vollständig gelingt, wird durch einen solchen Zusatz auch die Viskosität des betreffenden Produktes verändert und die Stabilität verringert.

Eine weitere Aufgabe war daher, all diesen den Nachteilen des Standes der Technik Abhilfe zu schaffen. Insbesondere sollten Produkte mit verringerter Klebrigkeit bzw. Schmierigkeit zur Verfügung gestellt werden. Produkte auf dem Gebiete der pflegenden Kosmetik, der dekorativen Kosmetik und der pharmakologischen Galenik sollten gleichermaßen von den geschilderten Nachteilen des Standes der Technik befreit werden.

Weiterhin war es eine Aufgabe der Erfindung, kosmetische Grundlagen für kosmetische Zubereitungen zu entwickeln, die sich durch gute Hautverträglichkeit auszeichnen.

Ferner war eine Aufgabe der vorliegenden Erfindung, Produkte mit einer möglichst breiten Anwendungsvielfalt zur Verfügung zu stellen. Beispielsweise sollten Grundlagen für Zubereitungsformen wie Reinigungsemulsionen, Gesichts- und Körperpflegezubereitungen, aber auch ausgesprochen medizinisch-pharmazeutische Darreichungsformen geschaffen werden, zum Beispiel Zubereitungen gegen Akne und andere Hauterscheinungen.

Als sogenannte "High Internal Phase"- Emulsionen werden nach K. J. Lissant: *The Geometry of High-Internal-Phase-Ratio Emulsions;* Journal of Colloid and Interface Science 22, 462-468 (1966) Emulsionen mit einer inneren Phase von mehr als 70 % definiert. Die Herstellung stabiler, fließfähiger Wasser-in-Öl-Emulsionen mit einem Wassergehalt von mehr als 70% stellt sich als sehr schwierig dar. Insbesondere sind "High Internal Phase"-W/O-Emulsionen mit einem sehr hohen Wassergehalt von mehr als 85% ("Very High Internal Phase"-W/O-Emulsionen) nicht zugänglich.

Die üblicherweise bei Wasser-in-Öl-Emulsionen angewandte Technik der Variation des Phasen-Volumen-Verhältnisses (d.h. Einarbeitung höherer Mengen an flüssigen Lipiden) kann, auf Grund des niedrigen Lipidanteils bei "High Internal Phase"-W/O-Emulsionen nur bedingt, bei "Very High Internal Phase"-W/O-Emulsionen) überhaupt nicht genutzt werden. Es sind daher nur Wasser-in-Öl-Emulsionen mit einer festen bis halbfesten Konsistenz zugänglich. Auch die Verwendung von polaren Lipiden, durch die üblicherweise niedrigviskosere Wasser-in-Öl-Emulsionen erhalten werden, führt nicht zum gewünschten Erfolg.

Überraschend hat sich gezeigt, daß Wasser-in-Öl-Emulsionen

(a) eines Gehaltes an Wasser und gegebenenfalls wasserlöslichen Substanzen von insgesamt mindestens 70 Gew.-%, und eines Gehaltes an Lipiden, Emulgatoren und lipophilen Bestandteilen von insgesamt höchstens 20% jeweils bezogen auf das Gesamtgewicht der Zubereitungen,

(b) mit einer Lipidphase deren Gesamtpolarität zwischen 20 und 45 mN/m liegt

(c) enthaltend wenigstens eine grenzflächenaktive Substanz, gewählt aus der Gruppe der Substanzen der allgemeinen Formel (I)

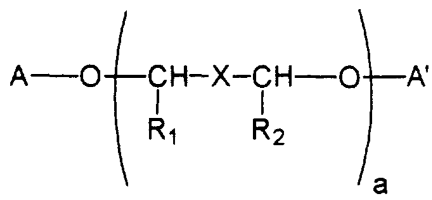

,

- wobei A und A' gleiche oder verschiedene organische Reste, gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema

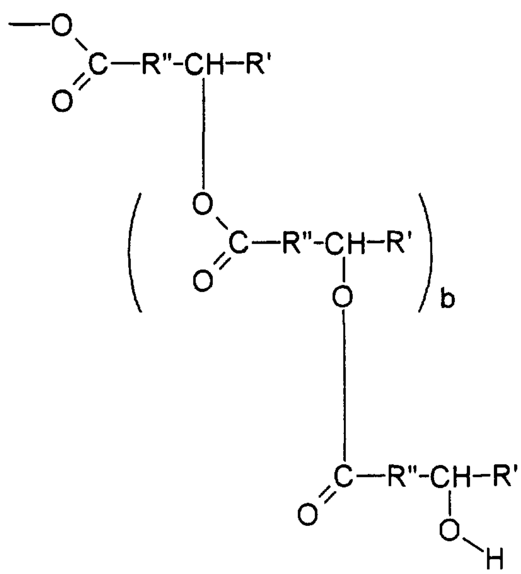

,

  wobei R' gewählt wird aus der Gruppe der verzweigten und unverzweigten Alkylgruppen mit 1 bis 20 Kohlenstoffatomen und R'' gewählt wird aus der Gruppe der verzweigten und unverzweigten Alkylengruppen mit 1 bis 20 Kohlenstoffatomen und b Zahlen von 0 bis 200 annehmen kann,
- a eine Zahl von 1 bis 100, vorzugsweise 2 bis 60, insbesondere 5 bis 40 darstellt,
- X eine Einfachbindung oder die Gruppe

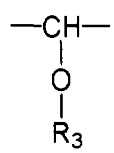

- darstellt,
- $R_1$ und $R_2$ unabhängig voneinander aus der Gruppe H, Methyl gewählt werden,
- $R_3$ gewählt wird aus der Gruppe H, sowie der verzweigten und unverzweigten, gesättigten und ungesättigten Alkyl- und Acylreste mit 1 - 20 Kohlenstoffatomen,

(d) enthaltend wenigstens eine Substanz, gewählt aus der Gruppe der nichtionischen Polymere den Nachteilen des Standes der Technik abhelfen .

Die Strukturformel ist nicht so zu interpretieren, daß durch den Index a alle in der Klammer repräsentierten Reste $R_1$, $R_2$ bzw $R_3$ im gesamten Molekül jeweils gleich sein müssen. Vielmehr können diese Reste in jedem der a

Fragmente

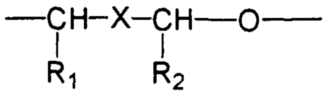

frei gewählt werden.

Es ist möglich und vorteilhaft, den Gesamtgehalt an Wasser und wasserlöslichen Substanzen der erfindungsgemäßen W/O-Emulsionen auf größer als 80 Gew.-%, insbesondere größer als 85 Gew.-% zu wählen, jeweils bezogen auf das Gesamtgewicht der Zubereitungen.

Ein Beispiel für besonders vorteilhaft im Sinne der vorliegenden Erfindung zu verwendende grenzflächenaktiven Substanzen ist das Polyethylenglycol-30-Dipolyhydroxystearat (PEG-30-Dipolyhydroxystearat), welches von der Gesellschaft ICI Surfactants unter der Warenbezeichnung ARLACEL® P135 verkauft wird.

Die Gesamtmenge an den erfindungsgemäß verwendeten grenzflächenaktiven Substanzen in den fertigen kosmetischen oder dermatologischen Zubereitungen wird vorteilhaft aus dem Bereich von 0,1 - 30 Gew.-%, bevorzugt 0,25 - 5,0 Gew.-% insbesondere 0,75 - 3,5 Gew.-% gewählt, bezogen auf das Gesamtgewicht der Zubereitungen.

Zwar ist bekannt, daß sich mit Emulgatoren der vorab beschriebenen Art W/O-Emulsionen erzeugen lassen. Dennoch konnte der bekannte Stand der Technik nicht den Weg zur vorliegenden Erfindung weisen.

Die erfindungsgemäß eingesetzten filmbildenden nichtionische Polymere können vorteilhaft aus der Gruppe der üblichen auf dem Gebiet der Kosmetik, insbesondere der Haarkosmetik eingesetzten Rohstoffe gewählt werden, aber auch Polymere für technische Anwendungszwecke, wie Beschichtungs- und Bindemittel.

Geeignete nichtionische Polymere sind beispielsweise

- Homopolymere des N-Vinylpyrrolidons, die als LUVISKOL® K-Typen von der Gesellschaft BASF bzw. PVP-K® -Typen von der Gesellschaft ISP mit verschiedenen mittleren Molmassen als Pulver oder in wässrigen bzw. wässrig/alkoholischen Lösungen angeboten werden.
  Bevorzugt wird hierbei der Typ Luviskol K30.
- Homopolymere des N-Vinylcaprolactam, z.B. von der Gesellschaft BASF unter den Handelsnamen LUVISKOL® Plus.
- Homopolymere des N-Vinylformamids.
- Copolymerisate aus N-Vinylpyrrolidon und Vinylacetat und/oder Vinylpropionat, die als LUVISKOL® VA-Typen von der Gesellschaft BASF bzw. PVP/VA ® -Typen von der Gesellschaft ISP in verschiedenen Konzentrationsverhältnissen als Pulver oder in wässrigen bzw. wässrig/alkoholischen Lösungen angeboten werden. Bevorzugt werden hier die Typen LUVISKOL® VA 37E und VA 64W
- Terpolymere aus N-Vinylpyrrolidon, Vinylacetat und Vinylpropionat, z.B. von der Gesellschaft BASF unter den Handelsnamen LUVISKOL® VAP 343.

Besonders bevorzugt werden hierbei die nichtionischen Polymere LUVISKOL® Plus und LUVISKOL® VA 64W.

Die Gesamtmenge an den erfindungsgemäß verwendeten nichtionischen Polymere in den fertigen kosmetischen oder dermatologischen Zubereitungen wird vorteilhaft aus dem Bereich von 0,1 - 30 Gew.-%, bevorzugt 0,25 - 5,0 Gew.-% insbesondere 0,75 - 3,5 Gew.-% gewählt, bezogen auf das Gesamtgewicht der Zubereitungen.

Überraschend hat sich insbesondere gezeigt, daß durch den Zusatz nichtionischer Polymere stabile, fließfähige "Very High Internal Phase Emulsions" hergestellt werden können, die über hervorragende sensorische Eigenschaften verfügen.

Es kann gegebenenfalls auch vorteilhaft sein, zusätzlich einen Gehalt an kationischen und/oder nichtionischen Polymeren zu verwenden, wobei die Gesamtmenge an solchen Polymeren in den fertigen kosmetischen oder dermatologischen Zubereitungen vorteilhaft aus dem Bereich von 0,1 - 30 Gew.-%, bevorzugt 0,25 - 5,0 Gew.-% insbesondere 0,75 - 3,5 Gew.-% gewählt wird, bezogen auf das Gesamtgewicht der Zubereitungen.

Im Rahmen der vorliegenden Offenbarung wird als Oberbegriff für Fette, Öle, Wachse und dergleichen gelegentlich der Ausdruck "Lipide" verwendet, wie dem Fachmanne durchaus geläufig ist. Auch werden die Begriffe "Ölphase" und "Lipidphase" synonym angewandt.

Öle und Fette unterscheiden sich unter anderem in ihrer Polarität, welche schwierig zu definieren ist. Es wurde bereits vorgeschlagen, die Grenzflächenspannung gegenüber Wasser als Maß für den Polaritätsindex eines Öls bzw. einer Ölphase anzunehmen. Dabei gilt, daß die Polarität der betreffenden Ölphase umso größer ist, je niedriger die Grenzflächenspannung zwischen dieser Ölphase und Wasser ist. Erfindungsgemäß wird die Grenzflächenspannung

als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen.

Die nachfolgende Tabelle 1 führt mittelpolare Lipide auf, die als Einzelsubstanzen oder auch im Gemisch untereinander erfindungsgemäß vorteilhaft sind. Die betreffenden Grenzflächenspannungen gegen Wasser sind in der letzten Spalte angegeben. Es ist jedoch auch vorteilhaft, Gemische aus höher- und niederpolaren und dergleichen zu verwenden, sofern gewährleistet ist, daß die Gesamtpolarität der Ölphase im beanspruchten Bereich liegt.

Tabelle 1

| **Handelsname** | INCI-Bezeichnung | **(mN/m)** |
|---|---|---|
| Isofol® 14 T | Butyl Decanol + Hexyl Decanol + Hexyl Octanol + Butyl Octanol | 27,6 |
| Isofol® 16 | Hexyl Decanol | 24,3 |
| Eutanol® G | Octyldodecanol | 24,8 |
| Cetiol® OE | Dicaprylyl Ether | 22,1 |
| Miglyol® 812 | Caprylic/Capric Triglyceride | 21,3 |
| Cegesoft® C24 | Octyl Palmitate | 23,1 |
| Isopropylstearat | Isopropyl Stearate | 21,9 |
| Estol® 1540 EHC | Octyl Octanoate | 30,0 |
| Finsolv® TN | $C_{12-15}$ Alkyl Benzoate | 21,8 |
| Cetiol® SN | Cetearyl Isonoanoate | 28,6 |
| Dermofeel® BGC | Butylene Glycol Caprylate/Caprate | 21,5 |
| Trivent® OCG | Tricaprylin | 20,2 |
| MOD | Octyldodeceyl Myristate | 22,1 |
| Cosmacol® ETI | Di-$C_{12-13}$ Alkyl Tartrate | 29,4 |
| Miglyol® 829 | Caprylic/Capric Diglyceryl Succinate | 29,5 |
| Prisorine® 2036 | Octyl Isostearate | 29,7 |
| Tegosoft® SH | Stearyl Heptanoate | 28,7 |
| Abil® Wax 9840 | Cetyl Dimethicone | 25,1 |
| Cetiol® LC | Coco-Caprylate/Caprate | 24,8 |
| IPP | Isopropyl Palmitate | 22,5 |
| Luvitol® EHO | Cetearyl Octanoate | 28,6 |
| Cetiol® 868 | Octyl Stearate | 28,4 |

Als Grenze, oberhalb derer eine Ölphase als "unpolar" gilt, werden allgemein ca. 30 mN/m angesehen.

Als besonders vorteilhafte unpolare Öle haben sich folgende, bei Raumtemperatur, flüssige unpolare Lipide erwiesen: Kohlenwasserstoffe (Mineralöle, Cyloparaffin, Polyisobutene, Polydecene), nicht ethoxylierte bzw. propopoxylierte Ether (Caprylylether / Cetiol OE) sowie Silikonöle (Dimethicone, Cylomethicone, Dimethiconol).

Nach obiger Definition der Polarität gelten Silikonöle nicht als unpolar sondern fallen in der Regel in die mittelpolare Region (typischerweise zwischen 20 und 30 mN/m).

Es ist erfindungsgemäß möglich, einen gewissen Anteil polarer Lipide, also solcher einer Polarität kleiner als 20 mN/m in der Lipidmischung zu dulden, keinesfalls soll dieser Anteil jedoch 25 Gew.-% übersteigen, beträgt bevorzugt weniger als 15 Gew.-% und soll im Idealfalle nicht mehr als ≤ 10 Gew.-%, bezogen auf die Gesamtlipidphase, betragen.

Gemäß der hiermit vorgelegten Lehre sind W/O-Emulsionen erhältlich, deren Viskosität bei 25° C kleiner als 5.000 mPa·s (= Millipascalsekunden) insbesondere kleiner als 4.000 mPa·s, bevorzugt kleiner als 3.500 mPa·s (HAAKE

Viscotester VT-02).

Vorteilhaft werden die erfindungsgemäßen Öle ebenfalls gewählt aus der Gruppe der Paraffinöle, Polyolefine sowie Vaseline (Petrolatum). Unter den Polyolefinen sind Polydecene und hydriertes Polyisobuten die bevorzugten Substanzen.

Die Ölphase kann im Sinne der vorliegenden Erfindung ferner - sofern die in den Patentansprüchen aufgeführten Merkmale beachtet werden - vorteilhaft Substanzen enthalten, gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, wie z.B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Gewünschtenfalls können in der Ölphase einzusetzende Fett- und/oder Wachskomponenten - als Nebenbestandteile in geringerer Menge - aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse.

Weitere vorteilhafte Fett- und/oder Wachskomponenten sind chemisch modifizierte Wachse und synthetische Wachse, wie beispielsweise die unter den Handelsbezeichnungen Syncrowax HRC (Glyceryltribehenat), Syncrowax HGLC ($C_{16-36}$ -Fettsäuretriglycerid) und Syncrowax AW 1C ($C_{18-36}$ -Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder $C_{30-50}$ -Alkyl Bienenwachs), Polyalkylenwachse, Polyethylenglykolwachse, aber auch chemisch modifizierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise $C_{20-40}$-Alkylstearat, $C_{20-40}$-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan.

Gewünschtenfalls können die Fett- und/oder Wachskomponenten sowohl einzeln als auch im Gemisch vorliegen.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als Lipidkomponente der Ölphase einzusetzen.

Von den Kohlenwasserstoffen sind Paraffinöl, hydrierte Polyolefine (z.B. hydriertes Polyisobuten) Squalan und Squalen vorteilhaft im Sinne der vorliegenden Erfindung zu verwenden.

Erfindungsgemäß besonders vorteilhaft sind solche Emulsionen, die dadurch gekennzeichnet sind, daß die Ölphase zu mindestens 50 Gew.-%, bevorzugt zu mehr als 75 Gew.-% aus mindestens einer Substanz, gewählt aus der Gruppe gewählt aus der Gruppe Vaseline (Petrolatum), Paraffinöl und Polyolefine, unter den letzteren bevorzugt: Polydecenen, besteht.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft kann Cyclomethicon (Octamethylcyclotetrasiloxan) eingesetzt werden. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B., Xanthangummi, Hydroxypropylmethylcellulose.

Ein besonderer Vorzug der vorliegenden Erfindung ist es, daß sie gestattet, hohe Konzentrationen an Polyolen, insbesondere Glycerin einzusetzen.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Carnosin und deren Derivate (z.B. Anserin), Carotinoide, Carotine (z.B. $\alpha$-Carotin, $\beta$-Carotin, $\Psi$-Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), femer (Metall)-Chelatoren (z.B. $\alpha$-Hydroxyfettsäuren, Palmitinsäure, Phytinsäure, Lactoferrin), $\alpha$-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Eine erstaunliche Eigenschaft der vorliegenden Erfindung ist, daß erfindungsgemäße Zubereitungen sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist dem Fachmann natürlich bekannt, daß kosmetische Zubereitungen zumeist nicht ohne die üblichen Hilfs- und Zusatzstoffe denkbar sind. Die erfindungsgemäßen kosmetischen und dermatologischen Zubereitungen können dementsprechend ferner kosmetische Hilfsstoffe enthalten, wie sie üblicherweise in solchen Zubereitungen verwendet werden, beispielsweise Konsistenzgeber, Stabilisatoren, Füllstoffe, Konservierungsmittel, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Viruzide, Wasser, Salze, antimikrobiell, proteolytisch oder keratolytisch wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch Elektrolyte.

Letztere können beispielsweise gewählt werden aus der Gruppe der Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte sind vorteilhaft, z.B. Lactate, Acetate, Benzoate, Propionate, Tartrate, Citrate, Aminosäuren, Ethylendiamintetraessigsäure und deren Salze und andere mehr. Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Besonders bevorzugt sind Kaliumchlorid, Kochsalz, Magnesiumsulfat, Zinksulfat und Mischungen daraus.

Mutatis mutandis gelten entsprechende Anforderungen an die Formulierung medizinischer Zubereitungen.

Die erfindungsgemäßen W/O-Emulsionen können als Grundlage für kosmetische oder dermatologische Formulierungen dienen. Diese können wie üblich zusammengesetzt sein und beispielsweise zur Behandlung und der Pflege der Haut und/oder der Haare, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika oder Dermatika üblichen Weise auf die Haut und/oder die Haare in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Die dünnflüssigen kosmetischen oder dermatologischen Mittel gemäß der Erfindung können beispielsweise als aus Aerosolbehältern, Quetschflaschen oder durch eine Pumpvorrichtung versprühbare Präparate vorliegen oder in Form einer mittels Roll-on-Vorrichtungen auftragbaren flüssigen Zusammensetzung, jedoch auch in Form einer aus normalen Flaschen und Behältern auftragbaren Emulsion.

Als Treibmittel für aus Aerosolbehältern versprühbare kosmetische oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UV-A-Filtersubstanz und/oder mindestens eine UV-B-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescrèmes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.:

- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethyl-hexyl)ester,
- Derivate des 1,3,5-Triazins, vorzugsweise 2,4,6-Trianilino-(p-carbo-2'-ethyl-1'-hexyloxy) -1,3,5-triazin.

Die Liste der genannten UVB-Filter, die in Kombination mit den erfindungsgemäßen Wirkstoffkombinationen

verwendet werden können, soll selbstverständlich nicht limitierend sein.

Es kann auch von Vorteil sein, erfindungsgemäße Lipodispersionen mit UVA-Filtern zu formulieren, die bisher üblicherweise in kosmetischen Zubereitungen enthalten sind. Bei diesen Substanzen handelt es sich vorzugsweise um Derivate des Dibenzoylmethans, insbesondere um 1-(4'-tertButylphenyl)-3-(4'-methoxyphenyl)propan-1,3-dion und um 1-Phenyl-3-(4'-isopropylphenyl)propan-1,3-dion.

Erfindungsgemäße kosmetische und dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks,' Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Als weitere Bestandteile können verwendet werden:

- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Erfindungsgemäße Zubereitungen können auch Wirkstoffe (eine oder mehrere Verbindungen) enthalten, welche gewählt werden aus der Gruppe: Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin $B_1$, das Vitamin $B_{12}$ das Vitamin $D_1$, aber auch Bisabolol, ungesättigte Fettsäuren, namentlich die essentiellen Fettsäuren (oft auch Vitamin F genannt), insbesondere die γ-Linolensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkemöl, Fischöle, Lebertran aber auch Ceramide und ceramidähnliche Verbindungen und so weiter. Vorteilhaft ist es auch, die Wirkstoffe aus der Gruppe der rückfettenden Substanzen zu wählen, beispielsweise Purcellinöl, Eucerit® und Neocerit®.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

| **Beispiel 1** | |
|---|---|
| | Gew.-% |
| PEG-30 Dipolyhydroxystearat | 1,5 |
| Isohexadekan | 12,0 |
| Isoeicosan | 5,0 |
| Glycerin | 3,0 |
| Polyvinylpyrrolidon | 1,25 |
| NaCl | 1,0 |
| Parfüm, Konservierungsmittel, Farbstoffe, Antioxidantien | q.s. |
| Wasser | ad. 100,00 |

| **Beispiel 2** | |
|---|---|
| | Gew.-% |
| PEG-30 Dipolyhydroxystearat | 1,0 |
| Dicaprylylether | 4,5 |
| Octylcocoat | 6,5 |

(fortgesetzt)

| **Beispiel 2** | |
|---|---|
| | Gew.-% |
| Glycerin | 3,0 |
| Hydroxypropyl Methylcellulose | 1,0 |
| $MgSO_4$ | 0,7 |
| Parfüm, Konservierungsmittel | |
| Farbstoffe, Antioxidantien | q.s. |
| Wasser | ad. 100,0 |

| **Beispiel 3 (W/O-Lotion)** | |
|---|---|
| | Gew.-% |
| PEG-30 Dipolyhydroxystearat | 2,0 |
| Isopropylstearat | 4,0 |
| $C_{12-15}$-Alkylbenzoat | 4,0 |
| Octylpalmitat | 4,0 |
| Glycerin | 3,0 |
| Polyvinylalkohol | 2,5 |
| NaCl | 1,0 |
| Parfüm, Konservierungsmittel | |
| Farbstoffe, Antioxidantien | q.s. |
| Wasser | ad. 100,0 |

| **Beispiel 4** | |
|---|---|
| | Gew.-% |
| PEG-30 Dipolyhydroxystearat | 1,5 |
| $C_{12-15}$ Alkylbenzoat | 10,0 |
| Glycerin | 3,0 |
| $MgSO_4$ | 0,7 |
| Polyether-1 | 0,5 |
| Parfüm, Konservierungsmittel, | |
| Farbstoffe, Antioxidantien | q.s. |
| Wasser | ad. 100,0 |

| **Beispiel 5** | |
|---|---|
| | Gew.-% |
| PEG-30 Dipolyhydroxystearat | 1,5 |
| Octylcocoat | 10,0 |
| Glycerin | 3,0 |
| Xanthan Gum | 0,75 |
| $MgSO_4$ | 0,7 |
| Parfüm, Konservierungsmittel, | |
| Farbstoffe, Antioxidantien | q.s. |
| Wasser | ad. 100,0 |

| Beispiel 6 | |
|---|---|
| | Gew.-% |
| PEG-30 Dipolyhydroxystearat | 1,5 |
| Isopropylstearat | 10,0 |
| Glycerin | 3,0 |
| Hydroxyethylcellulose | 1,0 |
| $MgSO_4$ | 0,7 |
| Parfüm, Konservierungsmittel, Farbstoffe, Antioxidantien | q.s. |
| Wasser | ad. 100,0 |

| Beispiel 7 | |
|---|---|
| | Gew.-% |
| PEG-30 Dipolyhydroxystearat | 2,0 |
| Dicaprylylcarbonat | 10,0 |
| Glycerin | 3,0 |
| Stärke | 1,25 |
| NaCl | 1,0 |
| Parfüm, Konservierungsmittel, Farbstoffe, Antioxidantien | q.s. |
| Wasser | ad. 100,0 |

| Beispiel 8 | |
|---|---|
| | Gew.-% |
| PEG-30 Dipolyhydroxystearat | 1,5 |
| Dicaprylylether | 10,0 |
| Glycerin | 3,0 |
| Guar Gummi | 1,0 |
| $MgSO_4$ | 0,7 |
| Parfüm, Konservierungsmittel, Farbstoffe, Antioxidantien | q.s. |
| Wasser | ad. 100,0 |

| Beispiel 9 | |
|---|---|
| | Gew.-% |
| PEG-30 Dipolyhydroxystearat | 1,5 |
| Butylenglycoldicaprylat/dicaprate | 10,0 |
| Glycerin | 3,0 |
| Glycerylmethacrylat | 0,75 |
| $MgSO_4$ | 0,7 |
| Parfüm, Konservierungsmittel, Farbstoffe, Antioxidantien | q.s. |
| Wasser | ad. 100,00 |

| Beispiel 10 | |
|---|---|
| | Gew.-% |
| PEG-30 Dipolyhydroxystearat | 1,5 |
| Dioctylcyclohexan | 10,0 |
| Glycerin | 3,0 |
| ,Glycerylmethacrylat | 1,0 |
| $MgSO_4$ | 0,7 |
| Parfüm, Konservierungsmittel, Farbstoffe, Antioxidantien | q.s. |
| Wasser | ad. 100,0 |

## Patentansprüche

**1.** Wasser-in-Öl- Emulsionen

(a) eines Gehaltes an Wasser und gegebenenfalls wasserlöslichen Substanzen von insgesamt mindestens 70 Gew.-%, und eines Gehaltes an Lipiden, Emulgatoren und lipophilen Bestandteilen von insgesamt höchstens 20% jeweils bezogen auf das Gesamtgewicht der Zubereitungen,
(b) mit einer Lipidphase deren Gesamtpolarität zwischen 20 und 45 mN/m liegt
(c) enthaltend wenigstens eine grenzflächenaktive Substanz, gewählt aus der Gruppe der Substanzen der allgemeinen Formel (I)

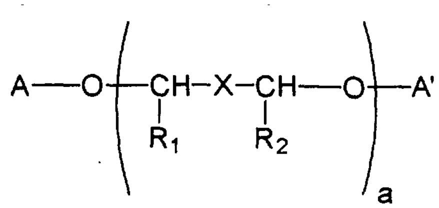

,

- wobei A und A' gleiche oder verschiedene organische Reste, gewählt aus der Gruppe der verzweigten und unverzweigten, gesättigten und ungesättigten Alkyl- und Acylreste und Hydroxyacylreste mit 10 - 30 Kohlenstoffatomen sowie femer aus der Gruppe der über Esterfunktionen miteinander verbundenen Hydroxyacylgruppen, nach dem Schema

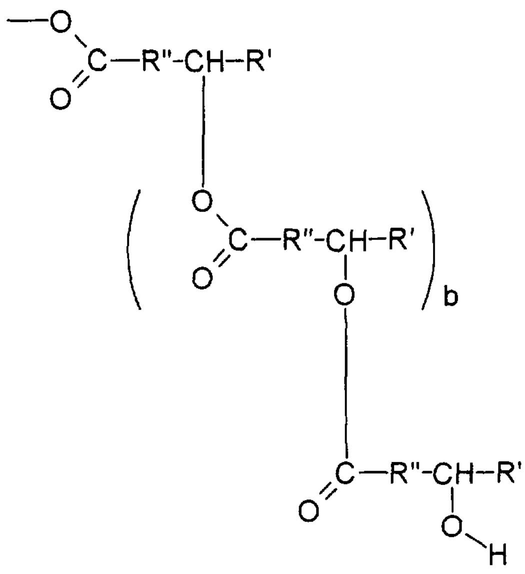

,

wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann,

- a eine Zahl von 1 bis 100, vorzugsweise 2 bis 60, insbesondere 5 bis 40 darstellt,
- X eine Einfachbindung oder die Gruppe

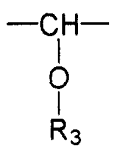

- darstellt,
- $R_1$ und $R_2$ unabhängig voneinander aus der Gruppe H, Methyl gewählt werden,
- $R_3$ gewählt wird aus der Gruppe H, sowie der verzweigten und unverzweigten, gesättigten und ungesättigten Alkyl- und Acylreste mit 1 - 20 Kohlenstoffatomen,

(d) enthaltend wenigstens eine Substanz, gewählt aus der Gruppe der nichtionischen Polymere

**2.** Emulsionen nach Anspruch 1, **dadurch gekennzeichnet, daß** ihr Gehalt an Wasser und wasserlöslichen Substanzen größer ist als 80 Gew.-%, insbesondere größer als 85 Gew.- %, jeweils bezogen auf das Gesamtgewicht der Zubereitungen.

**3.** Emulsionen nach Anspruch 1, **dadurch gekennzeichnet, daß** als grenzflächenaktive Substanz das Polyethylenglycol-30-Dipolyhydroxystearat gewählt wird.

**4.** Emulsionen nach Anspruch 1, **dadurch gekennzeichnet, daß** die Ölphase zu mindestens 50 Gew.-%, bevorzugt zu mehr als 75 Gew.-% aus mindestens einer Substanz, gewählt aus der Gruppe gewählt aus der Gruppe Vaseline (Petrolatum), Paraffinöl und Polyolefine, unter den letzteren bevorzugt: Polydecenen, besteht.

**5.** Emulsionen nach Anspruch 1, **dadurch gekennzeichnet, daß** sie 0,01 bis 10%, bevorzugt 0,25 - 1,25 % an nichtionischen Polymeren enthalten.

**6.** Emulsionen nach Anspruch 1, **dadurch gekennzeichnet, daß** das oder die nichtionischen Polymere gewählt werden aus der Gruppe der

Homopolymere des N-Vinylpyrrolidons

- Homopolymere des N-Vinylcaprolactam,
- Homopolymere des N-Vinylformamids
- Copolymerisate aus N-Vinylpyrrolidon und Vinylacetat und/oder Vinylpropionat,
- Terpolymere aus N-Vinylpyrrolidon, Vinylacetat und Vinylpropionat.